# EUROPEAN PATENT SPECIFICATION

(11) **EP 2 038 846 B1**
(45) Date of publication and mention of the grant of the patent: **28.04.2010**
(21) Application number: 07766774.9
(22) Date of filing: 18.06.2007
(51) Int. Cl.: G06T 7/60, G06T 7/00, A61B 6/12

(54) **MODEL-BASED DETERMINATION OF THE CONTRACTION STATUS OF A PERIODICALLY CONTRACTING OBJECT**
MODELLBASIERTE BESTIMMUNG DES KONTRAKTIONSSTATUS EINES PERIODISCH KONTRAHIERTEN OBJEKTS
DETERMINATION BASEE SUR UN MODELE DE L'ETAT DE CONTRACTION D'UN OBJET QUI SE CONTRACTE PERIODIQUEMENT

(30) Priority: 28.06.2006 EP 06116182
(43) Date of publication of application: 25.03.2009
(73) Proprietor: Philips Intellectual Property & Standards GmbH, 20099 Hamburg (DE); Koninklijke Philips Electronics N.V., 5621 BA Eindhoven (NL)
(72) Inventor: GROTH, Alexandra, NL-5656 AA Eindhoven (NL); ECK, Kai, NL-5656 AA Eindhoven (NL)
(86) International application number: PCT/IB2007/052322
(87) International publication number: WO 2008/001258

(56) References cited:
- US-A1- 2005 288 570
- US-A1- 2006 116 575
- US-B1- 6 493 575
- US-B1- 7 031 504
- US-B2- 6 849 048
- KARLSSON ET AL.: "Mitral valve opening in the ovine heart" AM J PHYSIOL HEART CIRC PHYSIOL, no. 274, 1998, pages 552-563, XP007903598
- SIERRA G ET AL: "Prototype of a fluoroscopic navigation systein to guide the catheter ablation of cardiac arrhythmias" PROCEEDINGS OF THE 25TH. ANNUAL INTERNATIONAL CONFERENCE OF THE IEEE ENGINEERING IN MEDICINE AND BIOLOGY SOCIETY. CANCUN, MEXICO, SEPT. 17, vol. VOL. 4 OF 4. CONF. 25, 17 September 2003 (2003-09-17), pages 138-141, XP010693099 ISBN: 0-7803-7789-3

## Description

The present invention relates to the field of digital image processing, in particular the invention relates to digital image processing for medical purposes, wherein a current contraction status of a periodically contracting object is determined.

Specifically, the present invention relates to a data processing device for determining the contraction status of a periodically contracting object, in particular for determining and visualizing the contraction status of a periodically moving object.

Further, the present invention relates to a catheterization laboratory for determining the contraction status of a periodically contracting object, in particular for determining and visualizing the contraction status of a periodically moving object, as well as to a computer-readable medium and to a program element having instructions for executing a method for determining the contraction status of a periodically contracting object.

Image guided medical and surgical procedures utilize patient images obtained prior to or during a diagnostic and/or treatment procedure to guide a physician performing the procedure.

At present, cardiac catheterization procedures are typically performed with the aid of two-dimensional (2D) fluoroscopic images. 2D fluoroscopic images taken intra-procedurally allow a physician to visualize the location of a catheter being advanced through cardiovascular structures.

In electrophysiological (EP) procedures, the position of a mapping catheter and/or an ablating catheter is often difficult to identify in relation to the individual patient's heart morphology because the heart is a non visible organ or only a shadow of the heart is visible in 2D fluoroscopic images. However, for performing a reliable EP procedure it is of great importance for the physician to have an idea where the catheter is currently located in relation to the patient's heart morphology. It is clear that the success of an ablation procedure strongly depends on the proper placement of the ablation catheter.

US 2006/0116575 A1 provides for methods and images for processing and/or superimposing a medical image of an anatomical body (e.g. a heart) with graphical information. Reference elements and/or reference catheters are placed in contact with the anatomical body. A physical structure within a navigational coordinate system is located using the reference elements and/or reference catheters.

EP 1 421 913 A1 discloses an image guided catheter navigation system for navigating through a region of a patient. The navigation system includes an imaging device, a tracking device, a controller and a display. The imaging device generates images of the region of the patient. The tracking device tracks the location of the catheter in the region of the patient. The controller superimposes an icon representing the catheter onto the images generated from the imaging device based upon the location of the catheter. The display shows the image of the region with the catheter superimposed onto the image at the current location of the catheter. However, this overlay between the image of the region and the image at the current location of the catheter becomes difficult in case the object under examination exhibits a constant movement. In particular in cardiac applications the effects of heart beat contractions complicate this overlay.

Although an estimation of the current contraction status of the heart from a 2D fluoroscopic image might be a possible solution, it has turned out that this solution fails in most cases because the X-ray shadow generated by the heart is often too faint to allow for a reliable measurement.

There may be a need for a precise determination of the contraction status of a periodically contracting object, which determination may be carried out in real time during an instrument such as a catheter is navigated through the object.

This need may be met by the subject matter according to the independent claims 1, 11 and 12 which define the invention. Advantageous embodiments of the present invention are described by the dependent claims 2-10.

1 # The invention relates to a data processing device adapted for performing a method for determining the contraction status of a periodically contracting object, in particular for determining and visualizing the contraction status of a periodically contracting object. The provided method comprises the steps of (a) determining a characteristic feature of the object for a series of different contraction statuses, wherein the characteristic feature is directly related to the current contraction status of the object, (b) evaluating a model representing the contraction status as a function of the characteristic feature, (c) acquiring a current dataset representing a current image of the object, (d) extracting the characteristic feature from the current image, (e) comparing the extracted characteristic feature to the model, and (f) obtaining the current contraction status.

The basic idea is to estimate the current contraction status of the periodically contracting object from current acquired images of the object by a model-based approach. Thereby, the object may be not or may be only partly visible in the current images. However, the characteristic feature representing an indication of the present contraction status is visible in the current image.

For the model-based estimation of the current contraction status of the object under examination and/or under treatment, a visible characteristic feature that is directly related to the more or less invisible contraction status is extracted from the current image of the object and subsequently compared to a model that gives the contraction status of the object in dependence on the characteristic feature. If the characteristic feature is determined e.g. by means of an a priori examination of the same object which is supposed to be examined and/or treated later on, an appropriate patient-individual model may be obtained. Thereby, the model may comprise an analytical function, which may be fitted to the determined characteristic features. However, the model may also be a simple table comprising a sequence of contraction statuses and the assigned characteristic features.

The provided method has the advantage that the current contraction status may be determined easily by a rather simple comparison between the characteristic feature and the a priori evaluated model. Thereby, the determination of the contraction status may be accomplished only with image-based information. Other information such as electrocardiogram based information is not necessary.

2# The invention further relates to the methods further comprising the steps of (a) acquiring a series of further current datasets each representing a further image of the object, (b) for each further current dataset, extracting the characteristic feature from the corresponding further current image, (c) comparing the further extracted characteristic features to the model, and (d) obtaining the corresponding further current contraction statuses. This has the advantage that a plurality of contraction statuses representing the full periodic cycle of the object under study may be obtained.

It has to be mentioned that one can employ two or even more different characteristic features or combinations of characteristic features in order to make the model evaluation procedure more stable. Thereby, an optimal model may be selected from a variety of different models, whereby the variety of different models are obtained from the different characteristic features or different feature combinations. This is in particular beneficial when the characteristic feature is the spatial distance between two or more reference objects and the exact location of these reference objects is not known precisely. Furthermore, the contraction status may be determined more precisely by registering two ore more model curves with each other.

3# The invention further relates to the characteristic feature being represented by the spatial difference between two reference points within the object. Preferably, the reference points are defined by so called reference catheters, which are inserted into the object under examination before employing a medical procedure and are in fix position during the whole procedure. Since they are attached to the contracting object their movement is characteristic for the contraction of the object. The reference catheters are visible on the current image.

In this respect it has to be pointed out that the characteristic feature may be determined by means of known automatic methods for extracting and tracking the positions of the reference objects respectively the reference catheters from current images.

In order to distinguish between catheters being moved within the object and reference catheters being firmly located in the standard positions e.g. for pacing experiments and EP recordings, the catheter movements are analyzed. By contrast to moving catheters such as e.g. ablation catheters, the reference catheters perform a regular periodic movement that is solely caused by patient's respiration and heart contraction and not by manual steering.

4# The invention further relates to the step of evaluating a model representing the contraction status as a function of the characteristic feature comprising using a deformable segmentation model of the object. This has the advantage that dedicated reference points from a prerecorded image may be automatically detected and propagated to a patient-individual segmented object anatomy. Thus, a model set of distance vs. contraction status curves that represent the distance between each two standard reference catheter positions in dependence of the object contraction status can be extracted from the segmented heart anatomy outlines.

5# The invention further relates to the periodically contracting object being a beating heart of a patient under examination. In this respect the method allows for realizing a precise catheter guiding with a model based estimation of the heart contraction by overlaying the correct non visible heart anatomy taken from prerecorded acquired images with the same contraction status from the same patient onto the current image showing the correct deformation of the contracted heart. Thereby, the model may be individually generated for the patient under study such that the described method allows for realizing a much more precise catheter guiding system than known catheter guiding systems.

It has to be mentioned that for an accurate catheter guiding not only the heart contraction caused by a heart beat but also the heart movement caused e.g. by the diaphragm pressure may be taken into account for the overlay. Thereby, the diaphragm pressure mainly causes a more or less rigid transformation of the heart. Hence, an image registration of the correctly chosen prerecorded image and the current image is additionally required. To this aim, well-known registration techniques exploiting the correspondences between reference points in a prerecorded image and in a current image can be used. Such registration techniques are often called point-to-point registrations.

6# The invention further relates to the step of acquiring a current dataset comprising the step of obtaining a two-dimensional (2D) image of the object and/or a three-dimensional (3D) image of the object. Current 2D images are preferably obtained by X-ray fluoroscopy. A 3D image may be obtained e.g. by means of known Magnetic Resonance (MR) techniques.

Fluoroscopic images may be obtained by means of standard X-ray equipment such as e.g. a C-arm being provided with an X-ray source and a corresponding X-ray detector. This has the advantage that a real time monitoring of the object under study may be realized without big computational costs. However, it has to be pointed out that also 3D MR images may be obtained in real time when modem MR systems are employed.

7# The invention also relates to the step of determining a characteristic feature of the object comprising the step of acquiring a series of different prerecorded datasets each representing a prerecorded image of the object at a different contraction status. As a result, full prerecord data sets for a series of different contraction statuses of the object under examination may be obtained. Thereby, a full representation of the contracting object may be obtained by means of standard medical equipment such as e.g. computed tomography (CT), magnetic resonance imaging (MRI), 3D rotational angiography (3D RA), fluoroscopy or any other image modality. Of course, by contrast to the current images the prerecord images comprise a visible representation of the whole periodically contracting object or at least of relevant parts of the periodically contracting object.

The generation of the prerecord images may be carried out by means of standard reconstruction methods, which are well known to persons skilled in the art.

It has to be mentioned that when the periodically contracting object is a beating heart, the heart phase may also be monitored e.g. by electrocardiogram apparatus. This allows for an in particular precise assignment of each prerecord image and the heart phase.

8# The invention also relates to the different prerecord datasets being acquired by means of computed tomography. Preferably, the different prerecord datasets are acquired by means of electrocardiogram (ECG) gated computed tomography. This may allow for a precise determination of the contraction state by means of known CT apparatuses. In particular an ECG gated CT allows for an easy and fast generation of precise prerecord images representing the different contraction statuses of the periodically contracting object.

9# The invention further relates to the step of determining a characteristic feature of the object further comprising the step of interpolating the contraction status and/or the corresponding characteristic feature between successive acquired prerecord datasets. Since in particular ECG gated CT acquisition provides only a limited number of contraction statuses of the heart, this may have the advantage that the number of supporting points of the full contraction course of the object respectively the heart contraction may be artificially increased. Thus, the overall model representing the time behavior of the contraction status may be obtained by an interpolation of the function representing the dependency of the distance between two reference points representing a possible characteristic feature from the current heart contraction status. Similarly, the missing corresponding heart anatomy outlines may be interpolated by exploiting the known surface point trajectories from the acquired CT data sets.

10# The invention also relates to the method further comprising the step of registering the current image to an arbitrary prerecord image in order to determine a foreshortening factor between the spatial distance of two reference points in the prerecord image and the corresponding spatial distance in the current image.

In this respect it has to be pointed out that the foreshortening factor is roughly the same for all contraction phases of a beating heart since the heart beat leads only to minor rotations and shifts which both can be neglected in a first approximation. This allows for a registration of any one of the prerecord images to the current image. Thereby, a model curve representing the temporal evolution of the contraction status may be calibrated. This allows for a more precise and more robust comparison of the extracted characteristic feature to the model. Therefore, the current contraction status may be determined even more precisely.

After the contraction status has been determined, the corresponding prerecord image can be registered to the current image. Of course, this registering may include also an automatic scaling of the model curve.

11# The invention also relates to the method further comprising the steps of (a) using the spatial distance of two reference points as the characteristic feature of the object and (b) calculating the spatial distance between the two reference points for a series of different contraction statuses by taking into account the foreshortening factor. This may provide the advantage that for each time instant of the periodically contracting object there may be calculated the distance between the two reference points resulting into a function representing the dependency between the distance between the two reference points and the time.

12# The invention further relates to the method further comprising the steps of (a) determining a plurality of foreshortening factors between spatial distances of a plurality of reference points among each other in the prerecord image and the corresponding spatial distances in the current image and (b) calculating the spatial distances between the plurality of reference points for a series of different contraction statuses by taking into account the foreshortening factors.

In other words, if more than two reference catheters are visible on the current image, distance vs. time curves for all possible catheter combinations may be extracted and the corresponding heart contraction statuses may be determined. By averaging all contraction statuses, the overall heart contraction status may be obtained more robustly. Thereby, a weighting factor can be introduced in the averaging process such that a stronger weight is assigned to pairs of reference catheters belonging to regions with the same instantaneous contraction status of the heart.

13# The invention also relates to the method further comprising the step of registering the calculated spatial distances representing the characteristic feature of the prerecord and the current image with each other in order to obtain the current contraction status. Preferably, a registering may be accomplished wherein a first curve depicting the spatial distance between two reference points vs. time obtained from the current image is registered with a variety of slightly different models, whereby the variety of different models are obtained from the different characteristic features or from different feature combinations. Preferably, the variety of slightly different models depict a plurality of corresponding spatial distances between respectively two reference points vs. the current heart contraction status.

The use of a variety of different model curves has the advantage that it is not necessary to determine which two out of a plurality of different reference points are visible in the current image. Since a model curve exists for each combination of reference points one simply has to select the appropriate model curve; which fits best to the time dependence of the characteristic feature being visible in the current images. This appropriate model curve is used in order to determine the current contraction status.

14# The invention also relates to the method further comprising the steps of (a) selecting one of the prerecord datasets representing the current contraction status and (b) registering this prerecord dataset with the current dataset. This provides the advantage that a real time imaging of the current scene e.g. an instrument being inserted into the object and the a priori information e.g. the contracted heart contour may be accomplished. Thereby, the current contraction status is identified from the characteristic feature being extracted from the current image.

It has to be pointed out that depending on the dimensionality of the prerecord dataset and the current dataset the registration procedure might be a 2D/2D, a 3D/2D or a 2D/3D registration.

15# The invention further relates to the method further comprising the steps of overlaying the prerecord image represented by the selected prerecord dataset and the current image represented by the current dataset. This has the advantage that an instrument being inserted into the object may be visualized within a prerecorded representation of the periodically contracting object. In other words, the current image is supplemented with an a priori non-visible information like the contour of the object. In case the object is a beating heart, the heart anatomy may be overlaid on the 2D fluoroscopic image that reflects the current contraction status of the heart.

In case a segmented heart anatomy outline is used for representing the object under examination, the calculated heart contraction status is used to pick the correct segmented heart anatomy outline that reflects the current heart contraction status and to overlay it on top of the current image, which is preferably a 2D X-ray fluoroscopy image. To this end, a registration between the current image and the prerecord image is performed by means of e.g. a point-to-point registration or a refinement of the initial registration of the current 2D image to an arbitrary volume of the 3D prerecord image and the result may be visualized in an appropriate way.

16# The invention also relates to the method further comprising the step of overlaying the current image represented by the current dataset with another image depicting the object in the current contraction status.

In other words, the current image is combined with a prerecorded image showing the object in the same contraction status as the current image. Thereby, the user may be provided with additional information regarding e.g. the morphology of the periodically contracting object. The prerecord images may be images obtained with any other imaging modality.

It has to be mentioned that the overlay procedure may be accomplished in various ways. Thereby, for instance whole images or segmented nodes may be overlaid with each other.

Preferably, the information regarding the contraction status may be exploited in a beneficial way by drawing these parts of the object, which are not visible in the current image but which are visible in a prerecord image representing the same contraction status, into the current image. Thereby, an a priori knowledge of e.g. the contour of the object under examination may be combined with the information of the current images, which are used for real time determining the contraction status of the periodically contracting object. This has the advantage that during an EP intervention of the beating heart one can monitor the exact spatial location of catheters with respect to the heart morphology in real time.

17# The invention also relates to a data processing device for determining the contraction status of a periodically contracting object, in particular for determining and visualizing the contraction status of a periodically contracting object. The data processing device comprises (a) a data processor, which is adapted for performing exemplary embodiments of the above-described method and (b) a memory for storing the evaluated model representing the contraction status as a function of the characteristic feature.

Preferably, the memory is also adapted for storing the acquired series of different prerecord datasets each representing a prerecord image of the object at different contraction statuses.

18 The invention also relates to a catheterization laboratory comprising the above-described data processing device.

19# The invention also relates to a computer-readable medium on which there is stored a computer program for determining the contraction status of a periodically contracting object, in particular for determining and visualizing the contraction status of a periodically contracting object. The computer program, when being executed by a data processor, is adapted for performing exemplary embodiments of the above-described method.

20# The invention also relates to a program element for determining the contraction status of a periodically contracting object, in particular for determining and visualizing the contraction status of a periodically contracting object. The program element, when being executed by a data processor, is adapted for performing exemplary embodiments of the above-described method.

The program element may be written in any suitable programming language, such as, for example, C++ and may be stored on a computer-readable medium, such as a CD-ROM. Also, the computer program may be available from a network, such as the World Wide Web, from which it may be downloaded into image processing units or processors, or any suitable computer.

The invention will be described in more detail hereinafter with reference to examples of embodiment.

Fig. 1 shows a flow chart on a method for determining and visualizing the contraction status of a beating heart.

Fig. 2a shows a curve depicting the time dependency of a distance between two reference catheters on a current 2D fluoroscopy image.

Fig. 2b shows a set of curves depicting the spatial distance between various reference catheters in the segmented heart anatomy as a function of the contraction status of the heart.

Fig. 3 shows the influence of the heart contraction on the reference catheter positions extracted from a 3D pre-interventional and a 2D live image.

Fig. 4 shows an image processing device for executing the preferred embodiment of the invention.

The illustration in the drawing is schematically.

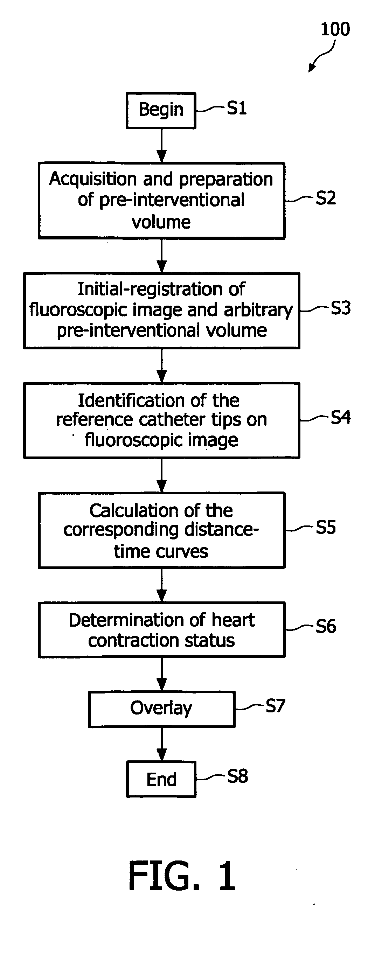

Figure 1 shows a flow chart 100 on a method for determining and visualizing the contraction status of a beating heart. The method starts with a step S1.

In step S2 an image of the object under examination is acquired in preparation for performing an electrophysiological (EP) procedure representing a minimal invasive treatment within the beating heart. Thereby, the object is examined by means of a known electrocardiogram (ECG) gated computed tomography (CT) system. As a result, pre-interventional three-dimensional (3D) data sets for a series of different contraction statuses of the heart are obtained. The pre-interventional 3D data sets are also called prerecord datasets.

By using a deformable segmentation model that comprises standard reference catheter positions, the dedicated points are propagated to a patient-individual segmented heart anatomy. Thus, a model set of distance vs. contraction status curves, that give the distance between each two standard reference catheter positions in dependence on the heart contraction status, can be extracted from the segmented heart anatomy outlines.

However, since the ECG gated CT acquisition provides only a limited number of contraction statuses of the heart, the number of supporting points of the distance vs. heart contraction status curves are limited. Thus, the overall model set is obtained by an interpolation of the distance vs. heart contraction status curves. Similarly, the missing corresponding heart anatomy outlines are interpolated by exploiting the known surface point trajectories from the acquired 3D CT datasets.

In step S3 an initial registration of a two-dimensional (2D) fluoroscopic image and an arbitrary pre-interventional volume is performed. This of course requires the acquisition of a corresponding 2D X-ray image which might preferably be carried out by means of a known C-arm X-ray system. Thereby, a current fluoroscopic image is registered to the arbitrary pre-interventional volume in order to determine a foreshortening factor between the real reference catheter's distance in 3D and the reference catheter's 2D distance appearing on the corresponding fluoroscopic projection.

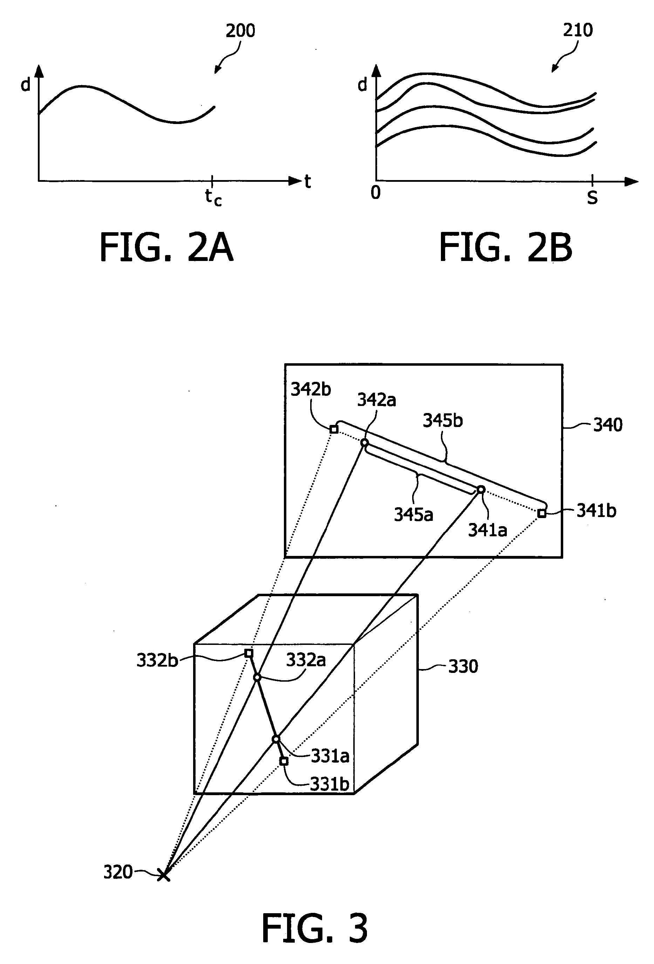

Fig. 3 shows the influence of the heart contraction on the reference catheter positions extracted from a 2D fluoroscopy image. Reference numeral 320 schematically denotes an X-ray source, which illuminates an arbitrary pre-interventional volume. According to the embodiment described here the pre-interventional volume is a patient's body. More specifically the pre-interventional volume is the beating heart of a patient. The pre-interventional volume is illustrated by a 3D image 330, which has been reconstructed by means of known CT reconstruction procedures. The circles 331a and 332a represent the position of a first reference catheter and of a second reference catheter respectively at a first heart phase. The squares 331b and 332b represent the position of the first reference catheter and of the second reference catheter respectively at a second heart phase. As one can see directly from Fig. 3, in between the first and the second heart phase the spatial distance between the two positions increases. This means, that the heart expands in between the first and the second heart phase.

In the corresponding 2D fluoroscopic image 340 representing a projection of the two reference catheters, the circles 341a and 342a represent the first reference catheter and the second reference catheter respectively at the first heart phase. Accordingly, the squares 342a and 342b represent the first reference catheter and the second reference catheter respectively at the second heart phase. Also within the 2D image 340, the distance 345a between the reference catheters 341 a and 342a at the first heart phase is smaller than the distance 345b between the reference catheters 34 1 b and 342b at the second heart phase. By comparing the 3D distance with the corresponding 2D distance between the catheter positions one can determine the foreshortening factor between the real reference catheter's distance in 3D and the reference catheter's 2D distance.

Is has to be mentioned that the foreshortening factor, which is determined in step S3, is roughly the same for all heart phases. This is based on the matter of fact that the heart beat leads only to minor rotations and minor shifts that can be neglected in a first approximation.

In step S4 the reference catheter tips are identified on the 2D fluoroscopic image. This can be done by known automatic methods for extracting the positions of reference catheters from 2D images, in particular from 2D fluoroscopic images.

In step S5 distance vs. time curves are calculated. Thereby, the foreshortening factor, which has been determined in step S3, is taken into account. The distance d between the reference catheters on the 2D fluoroscopic image is calculated for each time instant t resulting into the distance vs. time curve 200, which is depicted in Fig. 2a. Therein, t_{c} denotes the current time instant.

In order to identify the corresponding standard positions in the segmented heart anatomy all standard positions of the registered heart mesh are projected onto the fluoroscopic image. Thereby, the relevant distance vs. heart contraction curve can be identified.

Although the projected standard positions will probable be not completely congruent with the reference catheter's position on the current fluoroscopic image, the difference will be small and the distance to the surrounding standard reference points large enough to identify the correct standard positions in the segmented heart anatomy.

In order to distinguish between moving catheters being inserted into the beating heart and reference EP catheters being firmly located in the standard positions e.g. for pacing experiments and EP recordings, the catheter movements are analyzed. By contrast to moving catheters such as e.g. ablation catheters, the reference catheters perform a regular periodic movement that is solely caused by patient's respiration and heart contraction and not by manual steering.

If more than two reference catheters are visible on the 2D fluoroscopic image, the distance vs. time curves for all possible catheter combinations are extracted and the corresponding heart contraction statuses are determined. By averaging all contraction statuses, the overall heart contraction status is obtained. In doing so, a weighting factor can be introduced in the average process such that a stronger weight is assigned to pairs of reference catheters belonging to a regions with the same instantaneous contraction status of the heart.

In step S6 the current heart contraction status is determined. According to the embodiment described here, the current heart contraction status is obtained by registering the distance vs. time curve (see Fig. 2a) and the corresponding distance vs. heart contraction curves, which are depicted in Fig. 2b.

Fig. 2b shows a set 210 of curves depicting the spatial distance between various reference catheters in the segmented heart anatomy as a function of the contraction status of the heart. The set of curves results from the combination of all possible reference catheters.

In step S7, the calculated heart contraction status is used to pick the correct segmented heart anatomy outline that reflects the current heart contraction status and to overlay it on top of the 2D X-ray fluoroscopy image. To this end, a 2D/3D registration to the fluoroscopic image is performed and the result is visualized in an appropriate way by means of e.g. a point-to-point registration or a refinement of the initial registration of the current 2D image to an arbitrary volume of the 3D image.

Finally, the method ends with a step S8.

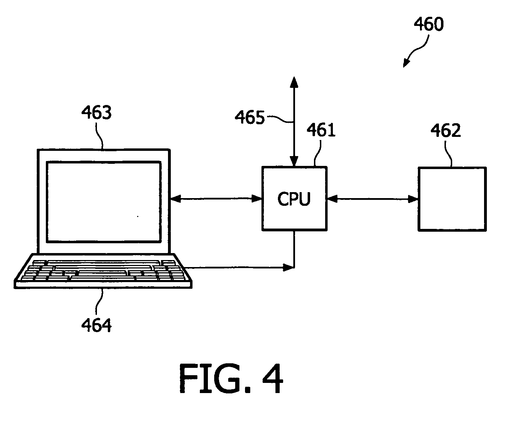

Fig. 4 depicts an exemplary embodiment of a data processing device 425 according to the present invention for executing a method as described. The data processing device 425 comprises a central processing unit (CPU) or image processor 461. The image processor 461 is connected to a memory 462 for temporally storing acquired or processed datasets. Via a bus system 465 the image processor 461 is connected to a plurality of input/output network or diagnosis devices, such as a CT scanner and a C-arm being used for 2D X-ray imaging. Furthermore, the image processor 461 is connected to a display device 463, for example a computer monitor, for displaying overlaid images by means of the above-described 2D/3D registration. An operator or user may interact with the image processor 461 via a keyboard 464 and/or any other output devices, which are not depicted in Fig. 4.

In order to recapitulate one can state:

It is described a model-based estimation of the current contraction status (S) of a periodically contracting object such as a beating heart. Thereby, a characteristic feature that is directly related to the contraction status (S) is extracted from a current image (340) and subsequently compared to a model that gives the contraction status (S) of the heart in dependence on the characteristic feature. Thereby, an appropriate patient-individual model may be obtained e.g. from an ECG gated pre-interventional data set. A potential characteristic feature is the distance between two arbitrary reference catheters (341a, 341b, 342a, 342b) that are usually in a fix known standard position during a whole electrophysiological intervention. The knowledge about the current contraction status may be used to combine the current image with other images depicting the object in the same contraction status. Thereby, image elements, which depend on the contraction status and which are not viewable in the current image, may be made visible by overlaying different images depicting the object in one and the same contraction status.

The scope of the invention is to be limited only by the appended claims, which define the invention.

It should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### LIST OF REFERENCE SIGNS:

- 100: flow chart
- S1: step 1
- S2: step 2
- S3: step 3
- S4: step 4
- S5: step 5
- S6: step 6
- S7: step 7
- S8: step 8
- 200: diagram depicting distance - time curve
- 210: diagram depicting set of distance - heart contraction curve
- d: distance between two reference catheter positions
- S: contraction status
- 320: radiation source
- 330: 3D image / reconstructed CT image
- 331a: reference catheter 1 (heart phase 1)
- 331b: reference catheter 1 (heart phase 2)
- 332a: reference catheter 2 (heart phase 1)
- 332b: reference catheter 2 (heart phase 2)
- 340: 2D image / fluoroscopic image
- 341 a: reference catheter 1 (heart phase 1)
- 341b: reference catheter 1 (heart phase 2)
- 342a: reference catheter 2 (heart phase 1)
- 342b: reference catheter 2 (heart phase 2)
- 345a: distance between reference catheters (heart phase 1)
- 345b: distance between reference catheters (heart phase 1)
- 460: data processing device
- 461: central processing unit / image processor
- 462: memory
- 463: display device
- 464: keyboard
- 465: bus system

## Claims

1. A data processing device (460) for determining and visualizing the contraction status (S) of a periodically contracting object of a human, the data processing device comprising
a memory (462) for storing an evaluated model representing the contraction status (S) as a function of a characteristic feature, and
a data processor (461), which is adapted for performing a method comprising the steps of
determining a characteristic feature of the object for a series of different contraction statuses (S), wherein the characteristic feature is directly related to the current contraction status (S) of the object,
evaluating a model representing the contraction status (S) as a function of the characteristic feature,
acquiring a current dataset representing a current image (340) of the object, extracting the characteristic feature from the current image (340),
comparing the extracted characteristic feature to the model, and
obtaining the current contraction status (S),
wherein the step of determining a characteristic feature of the object comprises the step of acquiring a series of different prerecord datasets each representing a prerecord image (330) of the object at a different contraction status (S),
wherein the method further comprises the step of registering the current image to an arbitrary prerecord image in order to determine a foreshortening factor between the spatial distance of two reference points in the prerecord image and the corresponding spatial distance in the current image.

2. The data processing device according to claim 1, wherein the method further comprises the steps of
acquiring a series of further current datasets each representing a further current image (340) of the object,
for each further current dataset, extracting the characteristic feature from the corresponding further current image (340),
comparing the further extracted characteristic features to the model, and
obtaining corresponding further current contraction statuses (S).

3. The data processing device according to claim 1, wherein the step of determining a characteristic feature of the object further comprises the step of
interpolating the contraction status (S) and/or the corresponding characteristic feature between successive acquired prerecord datasets.

4. The data processing device according to claim 1, wherein the method further comprises the steps of
using the spatial distance of two reference points as the characteristic feature of the object and
calculating the spatial distance between the two reference points for a series of different contraction statuses by taking into account the foreshortening factor.

5. The data processing device according to claim 1, wherein the method further comprises the steps of
determining a plurality of foreshortening factors between spatial distances of a plurality of reference points among each other in the prerecord image and the corresponding spatial distances in the current image and
calculating the spatial distances between the plurality of reference points for a series of different contraction statuses (S) by taking into account the foreshortening factors.

6. The data processing device according to claim 5, wherein the method further comprises the step of
registering the calculated spatial distances with each other.

7. The data processing device according to claim 1, wherein the method further comprises the steps of
selecting one of the prerecord datasets representing the current contraction status (S) and
registering this prerecord dataset with the current dataset.

8. The data processing device according to claim 7, wherein the method further comprises the step of
overlaying the prerecord image represented by the selected prerecord dataset and the current image represented by the current dataset.

9. The data processing device according to claim 7, wherein the method further comprises the step of
overlaying the current image represented by the current dataset with another image depicting the object in the current contraction status.

10. A catheterization laboratory comprising
a data processing device (460) according to claim 1.

11. A computer-readable medium on which there is stored a computer program for determining the contraction status (S) of a periodically contracting object of a human, in particular for determining and visualizing the contraction status (S) of a periodically contracting object, the computer program, when being executed by a data processor (461), is adapted for performing the method as set forth in claim 1.

12. A program element
for determining the contraction status (S) of a periodically contracting object, in particular for determining and visualizing the contraction status (S) of a periodically contracting object of a human, the program element, when being executed by a data processor (461), is adapted for performing the method as set forth in claim 1.

## Patentansprüche

1. Datenverarbeitungsvorrichtung (460) zum Bestimmen und Visualisieren des Kontraktionsstatus (S) eines periodisch kontrahierenden Objekts eines Menschen, wobei die Datenverarbeitungsvorrichtung Folgendes umfasst:
einen Speicher (462) zum Speichern eines evaluierten Modells, das den Kontraktionsstatus (S) als eine Funktion eines charakteristischen Merkmals darstellt, und
einen Datenprozessor (461), der vorgesehen ist, um ein Verfahren mit den folgenden Schritten durchzuführen:
Bestimmen eines charakteristischen Merkmals des Objekts für eine Reihe von unterschiedlichen Kontraktionszuständen (S), wobei das charakteristische Merkmal in direktem Zusammenhang mit dem aktuellen Kontraktionsstatus (S) des Objekts steht,
Evaluieren eines Modells, das den Kontraktionsstatus (S) als eine Funktion des charakteristischen Merkmals darstellt,
Erfassen eines aktuellen Datensatzes, der ein aktuelles Bild (340) des Objekts darstellt,
Extrahieren des charakteristischen Merkmals aus dem aktuellen Bild (340),
Vergleichen des extrahierten charakteristischen Merkmals mit dem Modell, und
Erlangen des aktuellen Kontraktionsstatus (S),
wobei der Schritt des Bestimmens eines charakteristischen Merkmals des Objekts den Schritt des Erfassens einer Reihe von unterschiedlichen zuvor aufgezeichneten Datensätzen umfasst, die jeweils ein zuvor aufgezeichnetes Bild (330) des Objekts bei einem anderen Kontraktionsstatus (S) darstellen,
wobei das Verfahren weiterhin den Schritt des Registrierens des aktuellen Bildes mit einem willkürlichen zuvor aufgezeichneten Bild umfasst, um einen Verkürzungsfaktor zwischen dem räumlichen Abstand von zwei Referenzpunkten in dem zuvor aufgezeichneten Bild und dem entsprechenden räumlichen Abstand im aktuellen Bild zu bestimmen.

2. Datenverarbeitungsvorrichtung nach Anspruch 1, wobei das Verfahren weiterhin die folgenden Schritte umfasst:
Erfassen einer Reihe von aktuellen Datensätzen, die jeweils ein weiteres aktuelles Bild (340) des Objekts darstellen,
für jeden weiteren aktuellen Datensatz Extrahieren des charakteristischen Merkmals aus dem entsprechenden weiteren aktuellen Bild (340),
Vergleichen der weiteren extrahierten charakteristischen Merkmale mit dem Modell, und
Erlangen entsprechender weiterer aktueller Kontraktionszustände (S).

3. Datenverarbeitungsvorrichtung nach Anspruch 1, wobei der Schritt des Ermittelns eines charakteristischen Merkmals des Objekts weiterhin den folgenden Schritt umfasst:
Interpolieren des Kontraktionsstatus (S) und/oder des entsprechenden charakteristischen Merkmals zwischen aufeinanderfolgenden erfassten zuvor aufgezeichneten Datensätzen.

4. Datenverarbeitungsvorrichtung nach Anspruch 1, wobei das Verfahren weiterhin die folgenden Schritte umfasst:
Verwenden des räumlichen Abstands von zwei Referenzpunkten als das charakteristische Merkmal des Objekts und
Berechnen des räumlichen Abstands zwischen den beiden Referenzpunkten für eine Reihe von unterschiedlichen Kontraktionszuständen durch Berücksichtigen des Verkürzungsfaktors.

5. Datenverarbeitungsvorrichtung nach Anspruch 1, wobei das Verfahren weiterhin die folgenden Schritte umfasst:
Ermitteln einer Vielzahl von Verkürzungsfaktoren zwischen räumlichen Abständen einer Vielzahl von Referenzpunkten untereinander in dem zuvor aufgezeichneten Bild und den entsprechenden räumlichen Abständen in dem aktuellen Bild und
Berechnen der räumlichen Abstände zwischen der Vielzahl von Referenzpunkten für eine Reihe von unterschiedlichen Kontraktionszuständen (S) durch Berücksichtigen der Verkürzungsfaktoren.

6. Datenverarbeitungsvorrichtung nach Anspruch 5, wobei das Verfahren weiterhin den folgenden Schritt umfasst:
Registrieren der berechneten räumlichen Abstände miteinander.

7. Datenverarbeitungsvorrichtung nach Anspruch 1, wobei das Verfahren weiterhin die folgenden Schritte umfasst:
Auswählen von einem der zuvor aufgezeichneten Datensätze, die den aktuellen Kontraktionsstatus (S) darstellen und
Registrieren dieses zuvor aufgezeichneten Datensatzes mit dem aktuellen Datensatz.

8. Datenverarbeitungsvorrichtung nach Anspruch 7, wobei das Verfahren weiterhin den folgenden Schritt umfasst:
Überlagern des durch den ausgewählten zuvor aufgezeichneten Datensatz dargestellten zuvor aufgezeichneten Bildes und des durch den aktuellen Datensatz dargestellten aktuellen Bildes.

9. Datenverarbeitungsvorrichtung nach Anspruch 7, wobei das Verfahren weiterhin den folgenden Schritt umfasst:
Überlagern des durch den aktuellen Datensatz dargestellten aktuellen Bildes mit einem anderen Bild, das das Objekt in dem aktuellen Kontraktionsstatus zeigt.

10. Katheterlabor mit
einer Datenverarbeitungsvorrichtung (460) nach Anspruch 1.

11. Computerlesbares Medium, auf dem ein Computerprogramm gespeichert ist zum Bestimmen des Kontraktionsstatus (S) eines periodisch kontrahierenden Objekts eines Menschen, insbesondere zum Bestimmen und Visualisieren des Kontraktionsstatus (S) eines periodisch kontrahierenden Objekts, wobei das Computerprogramm, wenn es durch einen Datenprozessor (461) ausgeführt wird, vorgesehen ist, um das Verfahren nach Anspruch 1 durchzuführen.

12. Programmelement
zum Bestimmen des Kontraktionsstatus (S) eines periodisch kontrahierenden Objekts, insbesondere zum Bestimmen und Visualisieren des Kontraktionsstatus (S) eines periodisch kontrahierenden Objekts eines Menschen, wobei das Programmelement, wenn es durch einen Datenprozessor (461) ausgeführt wird, vorgesehen ist, um das Verfahren nach Anspruch 1 durchzuführen.

## Revendications

1. Dispositif de traitement de données (460) pour déterminer et visualiser l'état de contraction (S) d'un objet d'un être humain qui se contracte périodiquement, le dispositif de traitement de données comprenant
une mémoire (462) pour stocker un modèle évalué qui représente l'état de contraction (S) en tant que fonction d'un élément caractéristique, et
un processeur de données (461), qui est apte à réaliser un procédé comprenant les étapes consistant à
déterminer un élément caractéristique de l'objet pour une série de différents états de contraction (S), dans lequel l'élément caractéristique est directement relié à l'état de contraction actuel (S) de l'objet,
évaluer un modèle qui représente l'état de contraction (S) en tant que fonction de l'élément caractéristique,
acquérir un ensemble de données actuel représentant une image actuelle (340) de l'objet,
extraire l'élément caractéristique de l'image actuelle (340),
comparer l'élément caractéristique extrait au modèle, et
obtenir l'état de contraction actuel (S),
dans lequel l'étape consistant à déterminer un élément caractéristique de l'objet comprend l'étape consistant à acquérir une série de différents ensembles de données préenregistrés, chacun représentant une image préenregistrée (330) de l'objet à un état de contraction différent (S),
dans lequel le procédé comprend en outre l'étape consistant à enregistrer l'image actuelle sur une image préenregistrée arbitraire en vue de déterminer un facteur de raccourcissement entre la distance spatiale de deux points de référence dans l'image préenregistrée et la distance spatiale correspondante dans l'image actuelle.

2. Dispositif de traitement de données selon la revendication 1, dans lequel le procédé comprend en outre les étapes consistant à
acquérir une série d'ensembles de données actuels supplémentaires, chacun représentant une image actuelle supplémentaire (340) de l'objet,
pour chaque ensemble de données actuel supplémentaire, extraire l'élément caractéristique de l'image actuelle supplémentaire correspondante (340),
comparer les éléments caractéristiques extraits supplémentaires au modèle, et
obtenir des états de contraction actuels supplémentaires correspondants (S).

3. Dispositif de traitement de données selon la revendication 1, dans lequel l'étape consistant à déterminer un élément caractéristique de l'objet comprend en outre l'étape consistant à
interpoler l'état de contraction (S) et/ou l'élément caractéristique correspondant entre des ensembles de données préenregistrés acquis successifs.

4. Dispositif de traitement de données selon la revendication 1, dans lequel le procédé comprend en outre les étapes consistant à
utiliser la distance spatiale entre deux points de référence en tant que l'élément caractéristique de l'objet et
calculer la distance spatiale entre les deux points de référence pour une série de différents états de contraction en prenant en compte le facteur de raccourcissement.

5. Dispositif de traitement de données selon la revendication 1, dans lequel le procédé comprend en outre les étapes consistant à
déterminer une pluralité de facteurs de raccourcissement entre des distances spatiales d'une pluralité de points de référence entre eux dans l'image préenregistrée et les distances spatiales correspondantes dans l'image actuelle et
calculer les distance spatiales entre la pluralité de points de référence pour une série de différents états de contraction (S) en prenant en compte les facteurs de raccourcissement.

6. Dispositif de traitement de données selon la revendication 5, dans lequel le procédé comprend en outre l'étape consistant à
enregistrer les distance spatiales calculées les unes avec les autres.

7. Dispositif de traitement de données selon la revendication 1, dans lequel le procédé comprend en outre les étapes consistant à
sélectionner un des ensembles de données préenregistrés représentant l'état de contraction actuel (S) et
enregistrer cet ensemble de données préenregistré avec l'ensemble de données actuel.

8. Dispositif de traitement de données selon la revendication 7, dans lequel le procédé comprend en outre l'étape consistant à
superposer l'image préenregistrée représentée par l'ensemble de données préenregistré sélectionné et l'image actuelle représentée par l'ensemble de données actuel.

9. Dispositif de traitement de données selon la revendication 7, dans lequel le procédé comprend en outre l'étape consistant à
superposer l'image actuelle représentée par l'ensemble de données actuel avec une autre image représentant l'objet dans l'état de contraction actuel.

10. Laboratoire de cathétérisme comprenant
un dispositif de traitement de données (460) selon la revendication 1.

11. Support lisible par ordinateur sur lequel il est stocké un programme informatique pour détermine l'état de contraction (S) d'un objet d'un être humain qui se contracte périodiquement, en particulier pour déterminer et visualiser l'état de contraction (S) d'un objet qui se contacte périodiquement, le programme informatique, lorsqu'il est exécuté par un processeur de données (461), est apte à réaliser le procédé selon la revendication 1.

12. Elément de programme
pour déterminer l'état de contraction (S) d'un objet qui se contracte périodiquement, en particulier pour déterminer et visualiser l'état de contraction (S) d'un objet d'un être humain qui se contracte périodiquement, l'élément de programme, lorsqu'il est exécuté par un processeur de données (461), est apte à réaliser le procédé selon la revendication 1.
